(19) Europäisches Patentamt
European Patent Office
Office européen des brevets

(11) Publication number: **0 304 802 B1**

(12) **EUROPEAN PATENT SPECIFICATION**

(45) Date of publication of patent specification: **31.03.93**

(51) Int. Cl.⁵: **A61K 33/30**, A61K 31/35, A61K 31/44, //(A61K33/30, 31:44,31:35,31:19),(A61K31/44, 31:19),(A61K31/35,31:19)

(21) Application number: **88113428.2**

(22) Date of filing: **18.08.88**

The file contains technical information submitted after the application was filed and not included in this specification

# (54) Pharmaceutical composition and the use thereof.

(30) Priority: **25.08.87 US 89271**

(43) Date of publication of application:
**01.03.89 Bulletin 89/09**

(45) Publication of the grant of the patent:
**31.03.93 Bulletin 93/13**

(84) Designated Contracting States:
**DE FR GB IT**

(56) References cited:
**US-A- 4 225 600**

**MEDECINE ET HYGIENE, vol. 41/1499, 8th January 1983, pages 24-30, Geneve, CH; P. LAUGLER et al.: "Dermatologie"**

(73) Proprietor: **Dr. Kübler GmbH**
**Lerchenfeldstrasse 20**
**W-8000 München 22(DE)**

(72) Inventor: **Kübler, Ulrich,Dr.**
**Lerchenfeldstr. 20**
**W-8000 Munich 22(DE)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81 (DE)**

EP 0 304 802 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a pharmaceutical composition comprising a chromene derivative and bivalent zinc as well as the use of the said active ingredients for the making of medicaments effective against dermatoses and for cosmetic purposes.

Since about 1965, chromene derivatives have been employed in the oral treatment of allergic asthma and rhinitis. Probably the best known compound in this field is cromoglycinic acid. Since then structurally related chromene compounds have been synthesized and partly introduced in therapy of allergic disorders, such chromenes are e.g. minocromil, nedocromil, terbucromil, proxicromil, ambicromil, isocromil and others.

More recently, such chromenes have also been used in topical applications. Thus sodium cromoglycate has been employed for the topical treatment of atopic eczema in children (Haider, S.A., British Medical Journal, i:1570 (1977)). However, sodium cromoglycate has also been found to be ineffective for topical treatment of atopic eczema in children (Thirumoorthy, T. et al, British Medical Journal, ii:500 (1978)). Topical treatment of atopic eczema with sodium cromoglycate has at best yielded only a statistically significant improvement of mild or moderately severe atopic eczema after nine weeks in actively treated patients (Ariyanayagam, M. et al, British Journal of Dermatology, 112:343 (1985)). Thus, previously known anti-eczema compositions for topical treatment of atopic eczema are not very effective for all degrees of atopic eczema, including severe atopic eczema and they are effective only after a prolonged period of treatment.

US-A-4 225 600 describes chromene derivatives as active ingredients for pharmaceutical compositions suitable for application to the skin.

Although zinc oxide has been known as an ingredient in ointments, pastes and powders, it has been known essentially for its effects as an astringent (The Merck Index, 8th Edition 1968). Zinc acetate has been found to influence favourably the effects of the macrolide antibiotic erythromycine in topical compositions for the treatment of acne (Zineryt - trademark of Roehm Pharma GmbH, Darmstadt). The application of such an antibiotic substance is not always desirable because of allergic reactions to it, for example in cases of pregnancy.

Accordingly, an object of the present invention is to provide an effective composition which is useful for the treatment of all degrees of dernatoses, including severe atopic eczema, and for cosmetic purposes and which is effective after a short period of treatment. According to a further object of the invention the composition shall be free of antibiotics or hormone substances.

Other objects of the present invention will be apparent from the detailed description of the invention provided hereinafter.

The above-described objects of the present invention have been met by a composition comprising a pharmaceutically effective amount of bivalent zinc in combination with at least one chromene derivative of the general formula I

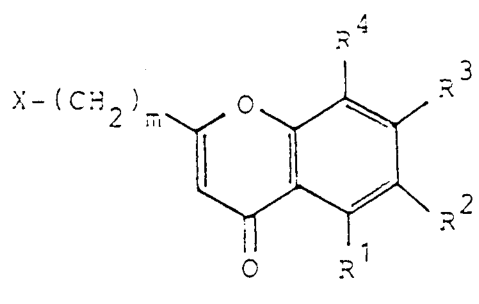

I

wherein

- m is 0 (zero), 1 or 2
- X is a carboxyl group or a phenyl radical which is optionally substituted by an alkyl group, having from 1 to 4 C atoms
- $R^1$ is hydrogen, a hydroxyl group or an optionally substituted radical having the general formula

$I^1$

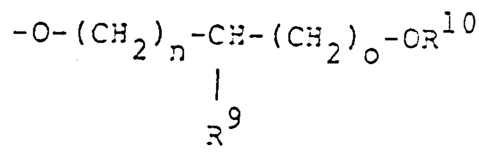

or

$I^2$

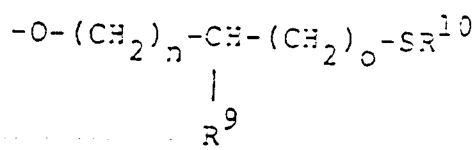

wherein n is 1 or 2, o is 0 (zero) or 1, $R^9$ is a hydrogen atom, a hydroxyl group or an alkyl radical having from 1 to 4 C atoms, $R^{10}$ is a hydrogen atom, an alkyl radical with 1 to 4 C atoms, a phenyl radical which is optionally substituted 1 to 3 times by an alkyl radical, a hydroxyl group;

$R^2$ is a hydrogen atom, a straight-chained or a branched alkyl radical with 1 to 4 C atoms or is a carboxyl group;

$R^3$ is a hydrogen atom or an alkyl group having from 1 to 3 C atoms or it has the meaning of $R^1$;

or

$R^2$ and $R^3$ together are Z, $Z_1$, $Z_2$,
or $Z_3$, where

Z =

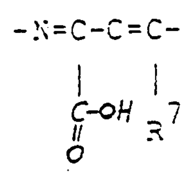

$Z_1$ =

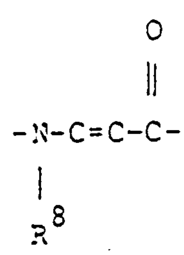

$Z_2$ = $-(CH_2)_4-$

$Z_3$ =

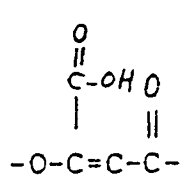

and form a carbocyclic or heterocyclic ring
wherein

$R^7$ is a hydrogen atom or an amino group which is optionally substituted by an alkyl radical having from 1 to 3 C atoms and $R^8$ is an alkyl group having from 1 to 3 C atoms;

$R^4$ is a hydrogen atom or a straight-chained or branched alkyl radical having from 1 to 4 C atoms;

or with chromoglycinic acid;
and pharmaceutically acceptable salts, esters and amides therefrom.

The preferred compounds useful in the composition of this invention are shown in the table as below:

Table

| active ingredient | m | n | o | X | $R^1$ | $R^9$ | $R^{10}$ | $R^2$ | $R^3$ | $R^4$ | $R^5$ | $R^6$ | $R^7$ | $R^8$ |
|---|---|---|---|---|---|---|---|---|---|---|---|---|---|---|
| cromoglycinic acid | 0 | 1 | 1 | $-COOH$ | $-O-(CH_2)_n-CH(R^9)-(CH_2)_o-OR^{10}$ | $-OH$ | 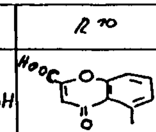 | $-H$ | $-H$ | $-H$ | — | — | — | — |
| MINOCROMIL | 0 | — | — | $-COOH$ | $-H$ | — | — | $-N=C(R^5)-C(R^6)=C(R^7)-$ | | $-C_3H_7$ | $-COOH$ | $-H$ | $-NH\cdot CH_3$ | — |
| FPL-52694 | 0 | 1 | 0 | $-COOH$ | $-O-(CH_2)_n-CH(R^9)-(CH_2)_o-OR^{10}$ | $-CH_3$ | $-H$ | $-H$ | $-H$ | $-C_3H_7$ | — | — | — | — |
| FPL-52757 | 0 | — | — | $-COOH$ | $-OH$ | — | — | $-CH_2CH_3$ | $-CH_2CH_3$ | $-CH_2CH_3$ | — | — | — | — |
| FPL-55712 | 0 | 1 | 1 | $-COOH$ | $-H$ | $-OH$ | 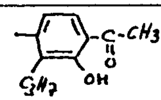 | $-H$ | $-O-(CH_2)_n-CH(R^9)-(CH_2)_o-OR^{10}$ | $-C_3H_7$ | — | — | — | — |
| NEDOCROMIL | 0 | — | — | $-COOH$ | $-H$ | — | — | $-N(R^8)-C(R^5)=C(R^6)-C(=O)-$ | | $-C_3H_7$ | $-COOH$ | $-H$ | — | $-C_2H_5$ |
| TERBUCROMIL | 0 | — | — | $-COOH$ | $-H$ | — | — | $-C(CH_3)_3$ | $-H$ | $-C(CH_3)_3$ | — | — | — | — |
| PROXICROMIL | 0 | — | — | $-COOH$ | $-OH$ | — | — | $-(CH_2)_4-$ | | $-C_3H_7$ | — | — | — | — |
| TEXACROMIL | 0 | 1 | 1 | $-COOH$ | $-O-(CH_2)_n-CH(R^9)-(CH_2)_o-SR^{10}$ | $-OH$ | $-CH_3$ | $-H$ | $-H$ | $-C_3H_7$ | — | — | — | — |
| FPL-59257 | 2 | 2 | 0 | $-COOH$ | $-H$ | $-H$ | 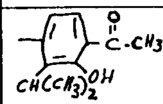 | $-H$ | $-O-(CH_2)_n-CH(R^9)-(CH_2)_o-OR^{10}$ | $-C_3H_7$ | — | — | — | — |
| AMBICROMIL | 0 | — | — | $-COOH$ | $-H$ | — | — | $-O-C(R^5)=C(R^6)-C(=O)-$ | | $-C_3H_7$ | $-COOH$ | $-H$ | — | — |
| ISOCROMIL | 0 | — | — | 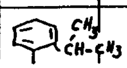 | $-H$ | — | — | $-COOH$ | $-H$ | $-H$ | — | — | — | — |

Compounds being especially preferred in the present compositions are those of the general formula

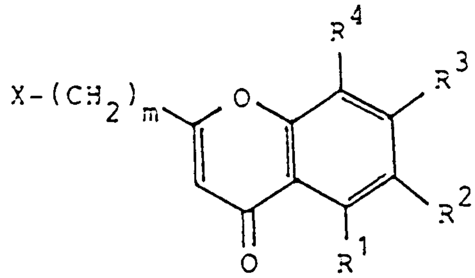

I

wherein m = 0, n = 1, o = 0 or 1 and X is a carboxyl group, the other radicals having the meaning as shown hereinbefore.

Furthermore, preferred compositions are those in which the chromene derivative is a compound of formula I wherein m = 0, $R^1$ is $I^1$ or $I^2$ (wherein n = 1, o = 0 or 1) and X is a carboxyl group and the other radicals have the meaning as shown hereinbefore.

The chromene derivative which is especially preferred in this invention is cromoglycinic acid and the pharmaceutically active salts, lower alkyl esters ($C_1$-$C_{10}$, particularly $C_1$-$C_4$) or amides which may be alkylated by $C_1$-$C_4$-alkyl thereof. Among the pharmaceutically preferred salts the alkali metal and alkaline earth metal salts can be mentioned. For some applications it may be possible also to use the zinc salts of cromoglycinic acid and, in a broader aspect, of the respective chromene derivative.

The above chromene compounds are known as is their synthesis so that no further discussion thereof is necessary.

The alkali metal when employed in the present invention is not critical thereto and can be any pharmaceutically acceptable alkali metal such as sodium, potassium, lithium, rubidium or combinations thereof. Sodium is the preferred alkali metal.

The alkaline earth metal when employed in the present invention is not critical thereto and can be any pharmaceutically acceptable alkaline earth metal such as calcium or magnesium. Magnesium is the preferred alkaline earth metal.

The amount of the chromene derivative is in a pharmaceutically or cosmetically effective amount which in general is from 1% to 25%, preferably from 2% to 10% by weight based on the total weight of the composition.

The specific bivalent zinc employed in the present invention is not critical thereto and can be in the form of bivalent zinc compounds such as zinc oxide, zinc lactate, zinc chloride, zinc carbonate, zinc sulfate, zinc acetate or combinations thereof. Zinc oxide is the preferred form of bivalent zinc employed in the present invention because it has a white colour and easily masks the skin well. If the particular form of bivalent zinc employed is highly acidic, pharmaceutically acceptable buffering agents can also be employed to adjust the pH to about physiological levels, i.e. about pH 5.0 to 7.7.

The bivalent zinc is also employed in an effective amount ranging from 1% to 25%, preferably from 4% to 10% by weight of bivalent zinc compound based on the total weight of the composition.

The composition of this invention is highly effective in the treatment of skin disorders in mammals (cats, horses, dogs) and particularly in man to any degree. Normally the composition will be applied as a pharmaceutical but also cosmetic applications may be possible. The skin disorders which can be successfully treated are dermatoses and these comprise particularly eczema including atopic eczema, psoriasis, dermatitic disorders due to allergic reaction (e.g. antibiotic, metals like Ni, organic chemicals), ulcers including colitis ulcerosa, urticaria, inflammatory skin conditions, e.g. due to Crohn's disease and others. The cosmetic applications may involve the treatment of acne, of wrinkles and of sun burns.

The compositions of this invention are normally applied by topical administration. Such topical applications the composition can be in form of solution, emulsion, suspension, ointment, cream, gel, lotion or spray. The use of emulsions, gels and ointments is generally preferred.

The preparation of such topical compositions is known to those skilled in the art. Some typical examples are given though below:

1. Emulsion

Oil phase

| | |
|---|---|
| 10 g | cetylstearyl alcohol |
| 10 g | cetylstearyl sulfonate |
| 10 g | olive oil |
| 5 g | wool wax |
| 10 g | zinc oxide |

Water phase

| | |
|---|---|
| 100 ml | double distilled water |
| 4 g | chromene compound |

2. Ointment

| | |
|---|---|
| 5 g | zinc acetate-dihydrate |
| 5 g | chromene compound |
| 25 g | paraffin oil |
| 60 g | paraffin wax |
| 5 g | wool fat |

3. Lotion

| | |
|---|---|
| 6 g | chromene compound |
| 3 g | zinc diacetate dihydrate |
| 60 g | ethanol (95%) |
| 20 g | di-isopropyl sebacate |
| 10.5 g | distilled water |
| 0.5 g | EDTA |

4. Cream

| | |
|---|---|
| 8 g | chromene compound |
| 5 g | wool alcohol |
| 25 g | solid paraffin mixture |
| 35 g | liquid paraffin |
| 7 g | zinc lactate |
| 20 g | purified water |

The topical compositions can be prepared using techniques known in the art. While topical compositions of varying types such as those described above can be utilized, the use of an oil-in-water or water-in-oil emulsion is often preferred.

For the preparation of oil based emulsions the oil used in the oil phase can be any pharmaceutically acceptable oil such as a vegetable oil, e.g. olive oil, soybean oil, jojoba oil, coconut oil and avocado oil, an animal oil such as whale oil and mink oil, or combinations thereof. Olive oil is the preferred oil because it is non-allergenic.

The water in the aqueous phase may be any pharmaceutically acceptable water such as sterile water, deionized water, double distilled water or tap water. Double distilled water is the preferred water because of the high degree of purity thereof.

If desired, additives such as preservatives, emulsifiers, emollients, biologically active substances such as naturally occurring flavonoides, sun screens, colorants, fragrances or other additives typically employed in topical compositions can be added to the composition of the present invention.

Examples of preservatives which can be employed include para-benzoic acid, sorbic acid and tocopherols. Para-benzoic acid can be added in an amount of from 0.5 to 3%, preferably from 1% to 1.5% by weight based on the total weight of the composition. Sorbic acid can be added in an amount of from 0.05 to 0.1% by weight based on the total weight of the composition. Tocopherols can be added in an amount of from 0.1 to 0.2% by weight based on the total weight of the composition. Generally, the preservatives are added to the aqueous phase. For solutions, sprays and lotions the use of pharmaceutically acceptable chelates and particularly of EDTA has been found to be useful for additional stabilization.

Examples of emulsifiers which can be employed in the present invention include cetylstearyl alcohol, cetylstearyl sulfonate, eucerin, Eucerit, Emulgol® (L. Givaudan & Co.), Emulgators 8077 and 8092 (Dragoco GmbH) and Emulgator oil/water (Ciba Geigy) or combinations thereof. Additional examples of emulsifiers which can be employed in the present invention are described in Kirk-Othmer Encyclopaedia of Chemical Technology 3rd Ed. (John Wiley & Sons, 1979, Vol. 7, pages 146-148). The emulsifiers can be added in an amount of from 10% to 20%, preferably from 12% to 15% by weight based on the total weight of the composition. Generally, the emulsifiers are added to the oil phase.

Examples of the emollients which can be employed in the present invention include wool wax, lecithin, cholesterin, eucerin, Eucerit and neatsfoot oil or combinations thereof. The emollients can be added in an amount of from 1.0% to 10%, preferably from 3.0% to 5.0% by weight based on the total weight of the composition. Generally, the emollients are added to the oil phase.

Examples of sun screens which can be employed in the present invention include triethanolamin, benzylanthranilate and 2-hydroxy-4′-methoxybenzophenone or combinations thereof. Additional examples of sun screens which can be employed in the present invention are described in Kirk-Othmer Encyclopaedia of Chemical Technology 3rd Ed. (John Wiley & Sons,1979, Vol. 7. pages 152-154). The sun screens can be added in an amount of from 0.5% to 6.0%, preferably from 1.0% to 5.0% by weight based on the total weight of the composition. Generally, the sun screens are added to the oil phase.

Colourants and fragrances as well as other additives can easily be added to the composition of the present invention in amounts conventionally employed as described in e.g. Kirk-Othmer Encyclopaedia of Chemical Technology 3rd Ed. (John Wiley & Sons,1979, Vol. 7, pages 143-176).

Emulsions of the present invention can be produced by first preparing an oil phase containing a water-insoluble bivalent zinc compound, such as zinc oxide, and then preparing an aqueous phase containing the chromene derivative, e.g. an alkali metal or alkaline earth metal cromoglycate. Bivalent zinc oxide can also be added to the water phase instead of the oil phase if desired. When the form of bivalent zinc is a water-soluble bivalent zinc compound, such as zinc chloride, it is added generally to the aqueous phase. The oil phase is then mixed with the water phase to achieve an oil-in-water emulsion or the water phase is added to the oil phase to achieve a water-in-oil emulsion.

The oil-in-water and water-in-oil emulsions can be prepared using conventional emulsification techniques such as a homogenizer, an emulsion mill and rollers. The particular size of the emulsion formed is not critical as long as the particle size is sufficient for the emulsion to be stable over a period of time.

The temperature for preparing the oil phase will be above the melting point of the oil but not above the temperature at which the chromene compound and additives are degraded. Generally, the temperature is 40 °C to 75 °C.

The composition of this invention is generally applied to the skin in an amount of 0.005 g to 0.03 g per square centimeter of skin, preferably 0.01 g to about 0.02 g per square centimeter of skin, although more or less can be employed as required by the patient's condition and the therapy desired.

Unless otherwise indicated all parts, percentages, and ratios are by weight.

Example

An oil phase containing the following components was prepared by admixing these components and melting the mixture at 75° C. Thereafter, the aqueous phase containing the following components was prepared by admixing these components at 40° C. Then the oil phase was mixed with the water phase to prepare an oil-in-water emulsion.

| Oil phase | |
|---|---|
| 10 g | cetylstearyl alcohol |
| 10 g | cetylstearyl sulfonate |
| 10 g | olive oil |
| 5g | wool wax |
| 10g | zinc oxide |

| Water phase | |
|---|---|
| 100 ml | double distilled water |
| 4 g | sodium cromoglycate |

The resulting emulsion was employed to treat 14 adults ranging from 20-60 years of age (12 females and 5 males) and 3 children ranging from the age of 9-12 years (2 females and 1 male).

At the start of the trial, the atopic eczema of each patient was assessed clinically into three groups (mild = I, moderately severe = II, severe = III) by recording the symptoms of itching, erythema, macules, lichenification, vesiculation, dryness and excoriation. The total severity was calculated by the addition of the score of each measurement over four main areas of eczema on the patients. During this trial, the patients did not receive any external medication, corticoids or antihistamines other than the use of the composition of the present invention.

The composition of the present invention was topically administered twice a day (in the morning and in the evening) in an amount of about 0.015 g per square centimeter. During the trial, one patient (a member of group II) was excluded because the patient began taking corticoids and another patient withdrew from the study because of dryness of the skin caused by application of the composition of the present invention (a member of group II).

During the study, the patients recorded the day when they were free of itching, vesiculation and pruritus and were evaluated every two days for the degree of severity of eczema. From the 15 patients which finished the study, 7 were in group I and 8 were in group II at the time of first clinical assessment. Two of the 7 of group I were free of symptoms at the third day of administering the composition of the present invention. Of the other 13 patients, 8 were free of symptoms on the seventh day and 5 on the tenth day. Of the 15 successfully treated patients, 4 needed no additional medication for weeks, 6 others administered the composition of the present invention occasionally to avoid excessive exacerbation and 5 administered the composition of the present invention routinely every day.

In previous trials of topical application of sodium cromoglycate alone employing proportionately the same amount of cromoglycate, a statistically significant improvement was not reached until 9 weeks after administration whereas using the composition of the present invention, improvement was observed after an average of only 7-10 days. Thus, the composition of the present invention is advantageous in that, due to the interaction of the alkali metal or alkaline earth metal cromoglycate and the bivalent zinc, it is effective in a shorter period of time than previous compositions employed for the treatment of atopic eczema and containing alkali metal or alkaline earth metal cromoglycate alone.

similar results are obtainable when replacing the cromoglycate by the chromene compounds as disclosed hereinbefore.

## Claims

**1.** Pharmaceutical composition comprising a pharmaceutically effective amount of bivalent zinc in combination with at least one chromene derivative of the general formula I

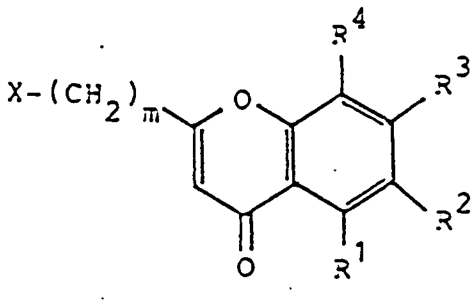

I

wherein

m is 0 (zero), 1 or 2

X is a carboxyl group or a phenyl radical which is optionally substituted by an alkyl group, having from 1 to 4 C atoms;

$R^1$ is hydrogen, a hydroxyl group or an optionally substituted radical having the general formula

$I^1$

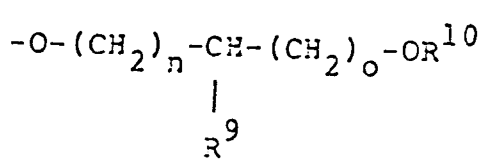

or

$I^2$

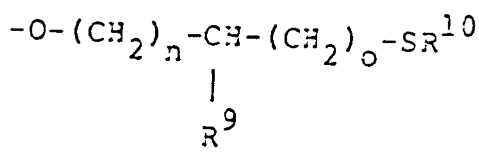

wherein n is 1 or 2, o is 0 (zero) or 1, $R^9$ is a hydrogen atom, a hydroxyl group or an alkyl radical having from 1 to 4 C atoms, $R^{10}$ is a hydrogen atom, an alkyl radical with 1 to 4 C atoms, a phenyl radical which is optionally substituted 1 to 3 times by an alkyl radical or a hydroxyl group;

$R^2$ is a hydrogen atom, a straight-chained or a branched alkyl radical with 1 to 4 C atoms or is a carboxyl group;

$R^3$ is a hydrogen atom or an alkyl group having from 1 to 3 C atoms of it has the meaning of $R^1$;

or

$R^2$ and $R^3$ together are Z, $Z_1$, $Z_2$,

or $Z_3$, when

Z =

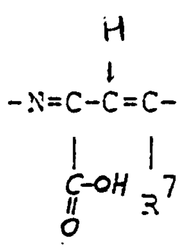

$Z_1$ =

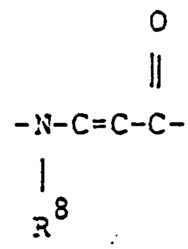

$Z_2$ = $-(CH_2)_4-$
$Z_3$ =

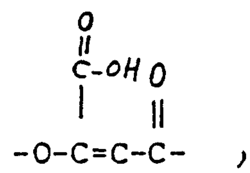

and form a carbocyclic or heterocyclic ring
wherein

- $R^7$ is a hydrogen atom or an amino group which is optionally substituted by an alkyl radical having from 1 to 3 C atoms and $R^8$ is an alkyl group having from 1 to 3 C atoms;
- $R^4$ is a hydrogen atom or a straight-chained or branched alkyl radical having from 1 to 4 C atoms;

or with chromoglycinic acid;
and pharmaceutically acceptable salts, esters and amides therefrom.

**2.** Composition according to claim 1, characterized in that the chromene derivative is a compound of formula I wherein m = 0, $R^1$ is $I^1$ or $I^2$ (wherein n = 1, o = 0 or 1) and X is a carboxyl group and the other radicals are as defined in in claim 1.

**3.** Composition according to claims 1 or 2, characterized in that the chromene derivative is cromoglycinic acid or a pharmaceutically acceptable salt thereof.

**4.** Composition according to any of the preceding claims in that it is in form appropriate for topical administration.

**5.** Composition according to any of the preceding claims, characterized in that said bivalent zinc is zinc oxide, zinc lactate, zinc chloride, zinc carbonate, zinc sulfate, zinc acetate or combinations thereof.

**6.** Composition according to any of the preceding claims, wherein said chromene derivative is present in an amount from 1% to 25% by weight based on the total weight of the composition.

**7.** Composition according to any of the preceding claims, wherein said bivalent zinc is present in an amount of from 1% to 25% by weight of bivalent zinc compound based on the total weight of the composition.

**8.** Topical composition according to any of the preceding claims containing cromoglycinic acid or a salt thereof and bivalent zinc in the form of an aqueous emulsion, suspension, ointment, cream, lotion or spray.

**9.** The use of a chromene derivative in combination with bivalent zinc according to any of the preceding claims for the preparation of a medicament effective against dermatoses, particularly atopic eczema.

**10.** The use of a chromene derivative in combination with bivalent zinc according to any of the preceding claims for the cosmetic treatment of the skin of mammals, notably that of man.

**11.** Composition according to any of claims 1 to 8 which is free of antibiotics or hormone substances.

**Patentansprüche**

**1.** Pharmazeutische Zusammensetzung umfassend eine pharmazeutisch wirksame Menge von zweiwertigem Zink in Kombination mit wenigstens einem Chromenderivat der allgemeinen Formel I

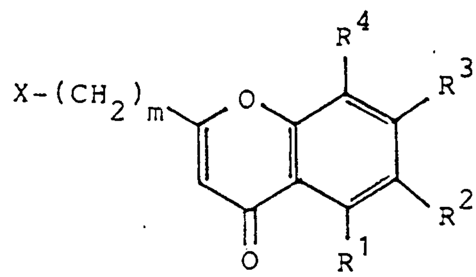

I

worin bedeuten

m 0 (Null), 1 oder 2

X eine Carboxylgruppe oder einen Phenylrest, der gewünschtenfalls durch eine Alkylgruppe mit 1 bis 4 C-Atomen substituiert ist,

$R^1$ Wasserstoff, eine Hydroxylgruppe oder ein gewünschtenfalls substituierter Rest der allgemeinen Formel

$I^1$ $$-O-(CH_2)_n-\underset{R^9}{\underset{|}{CH}}-(CH_2)_o-OR^{10}$$

oder

$I^2$ $$-O-(CH_2)_n-\underset{R^9}{\underset{|}{CH}}-(CH_2)_o-SR^{10}$$

worin n 1 oder 2 ist, o 0 (Null) oder 1 ist, $R^9$ ein Wasserstoffatom, eine Hydroxylgruppe oder ein Alkylrest mit 1 bis 4 C-Atomen ist, $R^{10}$ ein Wasserstoffatom, ein Alkylrest mit 1 bis 4 C-Atomen, ein Phenylrest, der gewünschtenfalls ein- bis dreimal durch einen Alkylrest substituiert ist, oder eine Hydroxylgrappe ist,

$R^2$ ein Wasserstoffatom, ein geradkettiger oder verzweigter Alkylrest mit 1 bis 4 C-Atomen, oder eine Carboxylgruppe;

$R^3$ ein Wasserstoffatom oder eine Alkylgruppe mit 1 bis 3 C-Atomen, oder die Bedeutung von $R^1$;

oder

$R^2$ und $R^3$ zusammen Z, $Z_1$, $Z_2$ oder $Z_3$ sind,

wobei

Z =

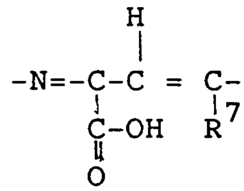

$Z_1$ =

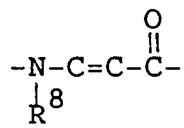

$Z_2$ = $-(CH_2)_4-$

$Z_3$ =

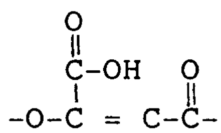

und einen carbozyklischen oder herozyklischen Ring bilden, worin bedeuten

$R^7$ ein Wasserstoffatom oder eine Aminogruppe, die gewünschtenfalls durch einen Alkylrest mit 1 bis 3 C-Atomen substituiert ist, und $R^8$ eine Alkylgruppe mit 1 bis 3 C-Atomen

$R^4$ ein Wasserstoffatom oder ein geradkettiger oder verzweigter Alkylrest mit 1 bis 4 C-Atomen;

oder mit Chromoglycinsäure

und pharmazeutisch annehmbare Salze, Ester und Amide davon.

2. Zusammensetzung gemäß Anspruch 1, dadurch **gekennzeichnet,** daß das Chromenderivat eine Verbindung der Formel I ist, worin m = 0, $R^1$ $I^1$ oder $I^2$ ist (worin n = 1, o = 0 oder 1) und X eine Carboxylgruppe ist, und die anderen Reste wie in Anspruch 1 definiert sind.

3. Zusammensetzung gemäß Ansprüchen 1 oder 2, dadurch **gekennzeichnet**, daß das Chromenderivat Chromoglycinsäure oder ein pharmazeutisch annehmbares Salz davon ist.

4. Zusammensetzung gemäß einem der vorhergehenden Ansprüche in einer für die topische Anwendung geeigneten Form.

5. Zusammensetzung gemäß einem der vorhergehenden Ansprüche, dadurch **gekennzeichnet,** daß das zweiwertige Zink Zinkoxid, Zinklaktat, Zinkchlorid, Zinkkarbonat, Zinksulfat, Zinkacetat oder eine Kombination davon ist.

6. Zusammensetzung gemäß einem der vorhergehenden Ansprüche, worin das Chromenderivat in einer Menge von 1 bis 25 Gew.-%, bezogen auf das Gesamtgewicht der Zusammensetzung, vorhanden ist.

7. Zusammensetzung gemäß einem der vorhergehenden Ansprüche, worin das zweiwertige Zink in einer Menge von 1 bis 25 Gew.-% einer zweiwertigen Zinkverbindung, bezogen auf das Gesamtgewicht der Zusammensetzung, vorhanden ist.

8. Topische Zusammensetzung gemäß einem der vorhergehenden Ansprüche, enthaltend Chromoglycinsäure oder ein Salz davon, und zweiwertiges Zink in Form einer wäßrigen Emulsion, Suspension, Salbe, Creme, Lotion oder als Spray.

9. Verwendung eines Chromenderivats in Kombination mit zweiwertigem Zink gemäß einem der vorhergehenden Ansprüche zur Herstellung eines Arzneimittels, das wirksam gegen Dermatosen, insbesondere atopische Ekzeme ist.

10. Verwendung eines Chromenderivats in Kombination mit zweiwertigem Zink gemäß einem der vorhergehenden Ansprüche für die kosmetische Behandlung der Haut von Säugern, insbesondere der von Menschen.

11. Zusammensetzung gemäß einem der Ansprüche 1 bis 8, die frei von Antibiotika oder Hormonsubstanzen ist.

**Revendications**

1. Composition pharmaceutique comprenant une quantité pharmaceutiquement efficace de zinc bivalent en association avec au moins un dérivé du chromène répondant à la formule générale I

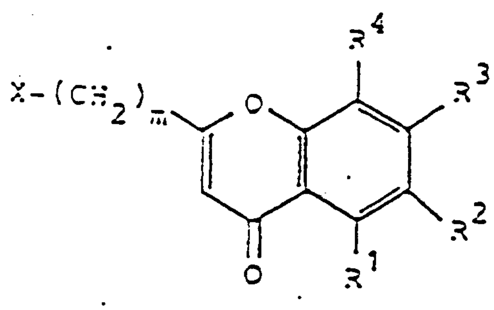

I

dans laquelle

m est 0 (zéro), 1 ou 2.

X est un groupe carboxyle ou un radical phényle , éventuellement substitué par un groupe alkyle ayant 1 à 4 atomes de carbone,

$R^1$ est un atome d'hydrogène, un groupe hydroxyle ou un radical éventuellement substitué répondant à la formule générale :

$I^1$

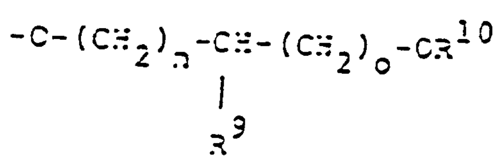

ou

$I^2$

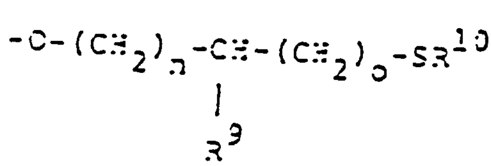

dans laquelle n est 1 ou 2, o est 0 (zéro) ou 1, $R^9$ est un atome d'hydrogène, un groupe hydroxyle ou un radical alkyle ayant 1 à 4 atomes de carbone, $R^{10}$ est un atome d'hydrogène, un radical alkyle ayant 1 à 4 atomes de carbone, un radical phényle éventuellement substitué, 1 à 3 fois,par un radical alkyle, ou un groupe hydroxyle;

$R^2$ est un atome d'hydrogène, un radical alkyle linéaire ou ramifié ayant 1 à 4 atomes de carbone ou un groupe carboxyle;

$R^3$ est un atome d'hydrogène ou un groupe alkyle ayant 1 à 3 atomes de carbone, ou bien il a la même signification que $R^1$;

ou

$R^2$ et $R^3$ sont ensemble Z, $Z_1$, $Z_2$, ou $Z_3$, où

Z =

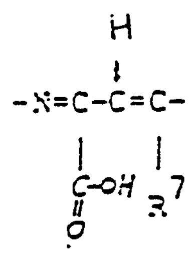

$Z_1$ =

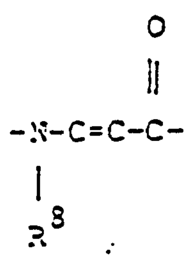

$Z_2$ = $-(CH_2)_4-$

$Z_3$ =

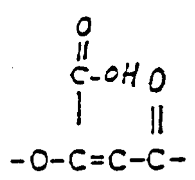
,

et forment un cycle carbocyclique ou hétérocyclique dans lequel

$R^7$ est un atome d'hydrogène ou un groupe amino qui est éventuellement substitué par un radical alkyle ayant 1 à 3 atomes de carbone, et $R^8$ est un groupe alkyle ayant 1 à 3 atomes de carbone;

$R^4$ est un atome d'hydrogène ou un radical alkyle linéaire ou ramifié ayant 1 à 4 atomes de carbone;

ou avec l'acide cromoglycinique;

et ses sels, esters et amides pharmaceutiquement acceptables.

**2.** Composition selon la revendication 1, caractérisée en ce que le dérivé du chromène est un composé répondant à la formule I dans laquelle m = 0, $R^1$ est $I^1$ ou $I^2$ (où n = 1, o = 0 ou 1) et X est un groupe carboxyle et les autres radicaux sont tels que définis dans la revendication 1.

**3.** Composition selon les revendications 1 ou 2, caractérisée en ce que le dérivé du chromène est l'acide cromoglycinique ou un de ses sels pharmaceutiquement acceptables.

4. Composition selon l'une quelconque des revendications précédentes, qui est sous une forme appropriée pour l'administration locale.

5. Composition selon l'une quelconque des revendications précédentes, caractérisée en ce que ledit zinc bivalent est l'oxyde de zinc, le lactate de zinc, le chlorure de zinc, le carbonate de zinc, le sulfate de zinc, l'acétate de zinc ou des associations de ceux-ci.

6. Composition selon l'une quelconque des revendications précédentes, dans laquelle ledit dérivé du chromène est présent dans une proportion de 1 % à 25 % en poids par rapport au poids total de la composition.

7. Composition selon l'une quelconque des revendications précédentes, dans laquelle ledit zinc bivalent est présent dans une proportion de 1 % à 25 % en poids de composé du zinc bivalent par rapport au poids total de la composition.

8. Composition locale selon l'une quelconque des revendications précédentes, contenant de l'acide cromoglycinique ou un de ses sels et du zinc bivalent sous la forme d'une émulsion aqueuse, d'une suspension, d'une pommade, d'une crème, d'une lotion ou d'une pulvérisation.

9. Utilisation d'un dérivé du chromène en association avec du zinc bivalent selon l'une quelconque des revendications précédentes pour la préparation d'un médicament efficace contre les dermatoses, en particulier l'eczéma atopique.

10. Utilisation d'un dérivé du chromène en association avec du zinc bivalent selon l'une quelconque des revendications précédentes pour le traitement cosmétique de la peau des mammifères, notamment de celle de l'homme.

11. Composition, selon l'une quelconque des revendications 1 à 8, qui est exempte d'antibiotiques ou d'hormones.